# EUROPEAN PATENT APPLICATION

(11) **EP 1 589 770 A2**
(43) Date of publication of application: **26.10.2005**
(21) Application number: 04255859.3
(22) Date of filing: 24.09.2004
(51) Int. Cl.: H04N 7/50, H04N 7/68, H04N 7/26

(54) **Apparatus and method for decoding a moving picture sequence**

(30) Priority: 20.04.2004 JP 2004124429
(71) Applicant: KABUSHIKI KAISHA TOSHIBA, Tokyo 105-8001 (JP)
(72) Inventor: Mirofumi, Mori Intellectual Property Division, Minato-ku Tokyo 105-8001 (JP)
(74) Representative: Shindler, Nigel

(57) **Abstract**

In an apparatus which decodes a moving picture, a coded stream is received, and a scene change I-frame, a P-frame and a refresh I-frame are discriminated. Each frame is decoded into frame picture data, and stored together with reference information in a buffer portion (5). If there is an error in the refresh I-frame when decoding the refresh I-frame, the P-frame picture data is set for restoration, the refresh I-frame is restored by making reference to the P-frame picture data and stored as reference frame picture data in a buffer area.

## Description

The present invention relates to a decoding apparatus which decodes coded moving picture information and a decoding method thereof, and more particularly to processing of coded moving picture information including an error.

As moving picture coding modes, there are MPEG-4 and H.264/MPEG-4 AVC (which will be referred to as H.264 hereinafter) developed from MPEG-4. H.264 is disclosed in, e.g., "Draft ITU-T Recommendation and Final Draft International Standard of Joint Video Specification", ITU-T Rec. H.264 ISO/IEC 14496-10 AVC, May 2003. This H.264 has been examined for adoption in ground-based digital broadcasting for mobiles.

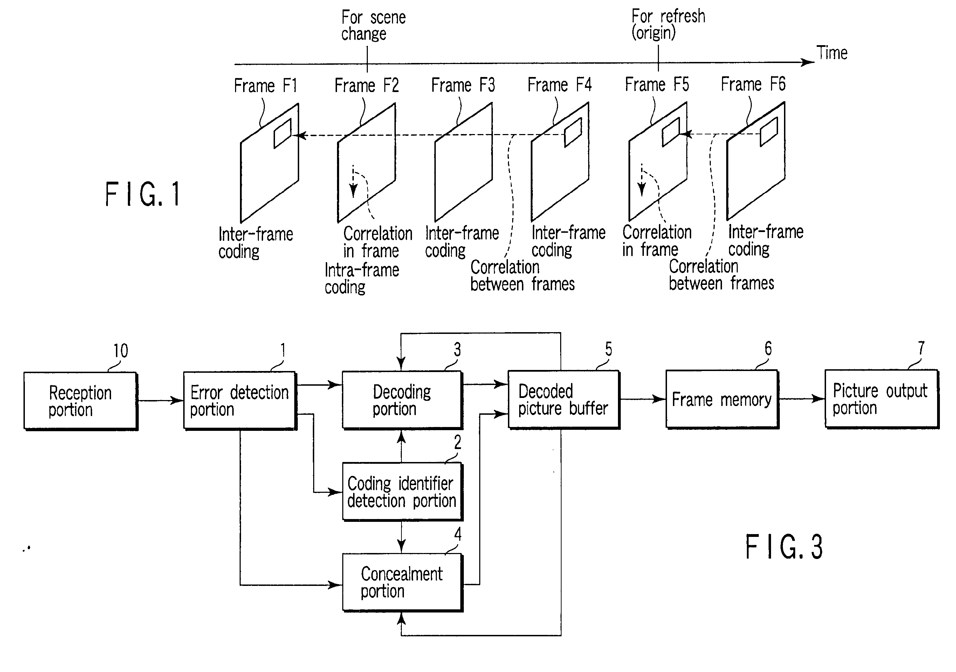

Frames of a moving picture in a coding mode defined by H.264 will now be described with reference to FIG. 1. In display of a moving picture, when frames corresponding to one screen constituting a moving picture are displayed based on a single frame step function, they are recognized as a moving picture in human eyes. That is, as shown in FIG. 1, frames F1, F2, F3, F4, F5 and F6 are transferred on the time base in the mentioned order, and these frames F1, F2, F3, F4, F5 and F6 are sequentially switched and displayed.

Since data of the moving picture has a large quantity information, it is coded and stored in a storage medium or provided to communication services as coded data. As this coding method, there is inter-frame coding by which moving picture data is coded by utilizing a correlation of moving picture data between frames and intra-frame coding by which moving picture data is coded by utilizing a correlation of moving picture data in a frame. In FIG. 1, the frames F1, F3, F4 and F6 correspond to frames subjected to inter-frame coding and the frames F2 and F5 correspond to frames subjected to intra-frame coding.

As general characteristics, a moving picture has a high correlation which is dependent on the time base and hence it has characteristics that a correlation between frames is high. In the inter-frame coding, a correlation on the time base is utilized and a frame is coded. In this inter-frame coding, a frame is coded by using a difference between the frame to be coded and a preceding frame, and an information quantity can be greatly reduced. When a picture content is largely switched, however, a difference between a frame to be coded and a preceding frame becomes large, and an information quantity reduction effect cannot be expected in some cases. Further, this coding requires a frame as an origin since a difference is used, and has characteristics that an error is propagated between frames on a decoding side when the error is mixed in inter-frame coded data.

On the other hand, the intra-frame coding is a coding method which is complete in a frame, and is also a coding method which performs coding by utilizing a correlation of adjacent picture portions in a frame. The intra-frame coding is provided with characteristics that a data quantity to be compressed is smaller than that in the inter-frame coding in a continuous sequence. Frames of the intra-frame coding are provided in order to realize two types of processing, i.e., scene change that a scene of a moving picture is switched and refresh that a moving picture is refreshed to a completely new moving picture frame.

The scene change corresponds to switching of a picture that a picture content is greatly switched as a drawback of the inter-frame coding, and a frame of the intra-frame coding is used since an information quantity reduction effect cannot be expected at the time of scene change.

As to the refresh, a frame of the intra-frame coding is periodically inserted in order to avoid error propagation between frames as a drawback of the inter-frame coding, and this frame of the intra-frame coding is an origin of the inter-frame coding. A frame of the inter-frame coding preceding a frame of the intra-frame coding is cleared as one for difference reference, thereby avoiding error propagation.

When a receiving side starts receiving with an arbitrary timing like a broadcasting service in particular, since there is no frame as an origin for a difference even if reception is started from a frame of the inter-frame coding, decoding of moving picture data of the frame cannot be immediately started. Therefore, in a decoder on the receiving side, a frame of the intra-frame coding is received, and this frame of the intra-frame coding is determined as an origin, thereby decoding subsequent frames of the inter-frame coding. In the ground-based digital broadcasting, a value of 500 milli-seconds is recommended as this refresh cycle in MPEG-2. In H.264, a value of 2 seconds (1.5 to 5 seconds) is recommended.

On a decoder side, as decoding processing, coded data is received, identification data of the inter-frame coding and the intra-frame coding are recognized, and decoding processing corresponding to the identification data is executed. In H.264, a plurality of reference frames can be utilized. These reference frames include one of picture information called "used for short term reference (reference)" and "used for long term reference (reference)", and they are stored in a decoded picture buffer (DPB). Then, reference is made to these reference frames, and the frame of the inter-frame coding is decoded. When decoding a frame of the refresh intra-frame coding, picture information of all reference frames stored in the decoded picture buffer is set as "unused for reference (non-reference)", and these frames are not used for subsequent decoding.

Furthermore, on the decoder side, as restoration processing when an error is mixed in coded data, concealment processing (restoration processing) utilizing a correlation between frames is executed in case of a frame of the inter-frame coding. In case of a frame of the intra-frame coding, there is carried out concealment processing (restoration processing) utilizing a correlation of adjacent pictures in the frame.

Adoption of H.264 has been examined for the ground-based digital broadcasting for mobiles. However, in case of utilizing broadcasting services in mobile communication terminals such as mobile phones, excellent reception of radio waves cannot be expected behind buildings or the like, and a possibility of mixing of errors at a high frequency is sufficiently expected.

It is an object of the present invention to provide a moving picture decoding apparatus which can execute simple concealment processing with respect to a moving picture transferred thereto when an error is generated in this moving picture and further improve a reproduced picture quality, and a decoding method thereof.

According to an aspect of the present invention, there is provided a moving picture decoding apparatus comprising:
a reception portion configured to receive a coded stream including a scene change I-frame coded in a frame, a first identifier for the scene change I-frame, a first P-frame coded between frames, a second identifier for the first P frame, a refresh I-frame coded in a frame following the first P-frame and a third identifier for the refresh I-frame;
a coding identifier detection portion configure to detect the first, second and third identifiers, and discriminate the scene change I-frame, the P-frame and the refresh I-frame;
a decoding portion configured to decode the scene change I-frame into first frame picture data in response to detection of the first identifier, decode the first P-frame into second frame picture data based on the I-frame picture data in response to detection of the second identifier, and decode the refresh I-frame into third frame picture data in response to the third identifier;
a buffer portion having first, second and third buffer areas in which the first, second and third frame picture data and first, second and third reference information are respectively stored; and
a restoration processing portion configured to set the second frame picture data for restoration if there is an error in the refresh I-frame when decoding the refresh I-frame, restores the refresh I-frame by making reference to the second frame picture data, and store it as the third frame picture data in the third buffer area.

Moreover, according to an aspect of the present invention, there is provided an apparatus which decodes a moving picture characterized by comprising:
a reception portion configured to receive a coded stream including a scene change I-frame coded in a frame, a first identifier for the I-frame, a first P-frame coded between frames, a second identifier for the first P frame, a refresh I-frame coded in a frame following the first P-frame and the first identifier for the I-frame;
a detection portion configured to detect the first and second identifiers and discriminate the I-frame and the P-frame, the detection portion measuring a cycle of the I-frame for an arbitrary number of times and judging the refresh I-frame based on the measured cycles;
a decoding portion configured to decode the scene change I-frame into first frame picture data in response to the first identifier, decodes the first P-frame into second frame picture data based on the I-frame picture data in response to detection of the second identifier, and decode the refresh I-frame into third frame picture data in response to the first identifier;
a buffer portion having first, second and third buffer areas in which the first, second and third frame picture data and first, second and third reference information are respectively stored; and
a restoration processing portion configured to set the second frame picture data for restoration if there is an error in the refresh I-frame when decoding the I-frame which is determined as the refresh I-frame in the detection portion, restores the refresh I-frame by making reference to the second frame picture data, and stores it as the third frame picture data in the third buffer area.

Further, according to yet another aspect of the present invention, there is provided a method for decoding a moving picture characterized by comprising:
receiving a coded stream including a scene change I-frame coded in a frame, a first identifier for the scene change I-frame, a first P-frame coded between frames, a second identifier for the first P-frame, a refresh I-frame coded in a frame following the first P-frame, and a third identifier for the refresh I-frame;
detecting the first, second and third identifiers, and discriminating the scene change I-frame, the P-frame and the refresh I-frame;
decoding the scene change I-frame into first frame picture data in response to detection of the first identifier, decoding the first P-frame into second frame picture data based on the I-frame picture data in response to detection of the second identifier, and decoding the refresh I-frame into third frame picture data in response to the third identifier;
storing the first, second and third frame picture data and first, second and third reference information in first, second and third buffer areas, respectively; and
setting the second frame picture data for restoration if there is an error in the refresh I-frame when decoding the refresh I-frame, restoring the refresh I-frame by making reference to the second frame picture data, and storing it as the first frame picture data in the third buffer area.

Furthermore, according to furthermore another aspect of the present invention, there is provided a method for decoding a moving picture characterized by comprising:
receiving a coded stream including a scene change I-frame coded in a frame, a first identifier for the I-frame, a first P-frame coded between frames, a second identifier for the first P-frame, a refresh I-frame coded in a frame following the first P-frame, and the first identifier for the I-frame;
detecting the first and second identifiers and discriminates the I-frame and the P-frame, measuring a cycle of the I-frame for an arbitrary number of times, and determining the refresh I-frame based on the measured cycles;
decoding the scene change I-frame into first frame picture data in response to detection of the first identifier, decoding the first P-frame into second frame picture data based on the I-frame picture data in response to detection of the second identifier, and decoding the refresh I-frame into third frame picture data in response to the first identifier;
storing the first, second and third frame picture data and first, second and third reference information in first, second and third buffer areas, respectively; and
setting the second frame picture data for restoration if there is an error in the refresh I-frame when decoding the I-frame determined as the refresh I-frame in the detection portion, restoring the refresh I-frame by making reference to the second frame picture data, and storing it as the third frame picture data in the third buffer area.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a schematic view illustrating a frame structure of general moving picture data with reference to a time base;
FIG. 2A is a schematic view showing a slice structure of a general frame;
FIG. 2B is a schematic view showing a structure of a general frame;
FIG. 3 is a block diagram schematically showing an apparatus which decodes a moving picture according to an embodiment of the present invention;
FIG. 4 is a flowchart showing steps which judges a type of a slice in a coding type detection portion depicted in FIG. 3;
FIG. 5 is a schematic view illustrating concealment processing with respect to each frame transferred on the time base in the apparatus depicted in FIG. 1;
FIG. 6 is a schematic view showing how the frames depicted in FIG. 5 are stored in a decoded picture buffer illustrated in FIG. 1 on the time base;
FIG. 7 is a flowchart showing a control operation in the decoded picture buffer depicted in FIG. 1;
FIG. 8 is a flowchart illustrating an operation of the decoding apparatus depicted in FIG. 1; and
FIG. 9 is a flowchart illustrating an operation of an apparatus which decodes a moving picture according to another embodiment of the present invention.

A moving picture decoding apparatus according to an embodiment of the present invention will now be described hereinafter with reference to the accompanying drawings.

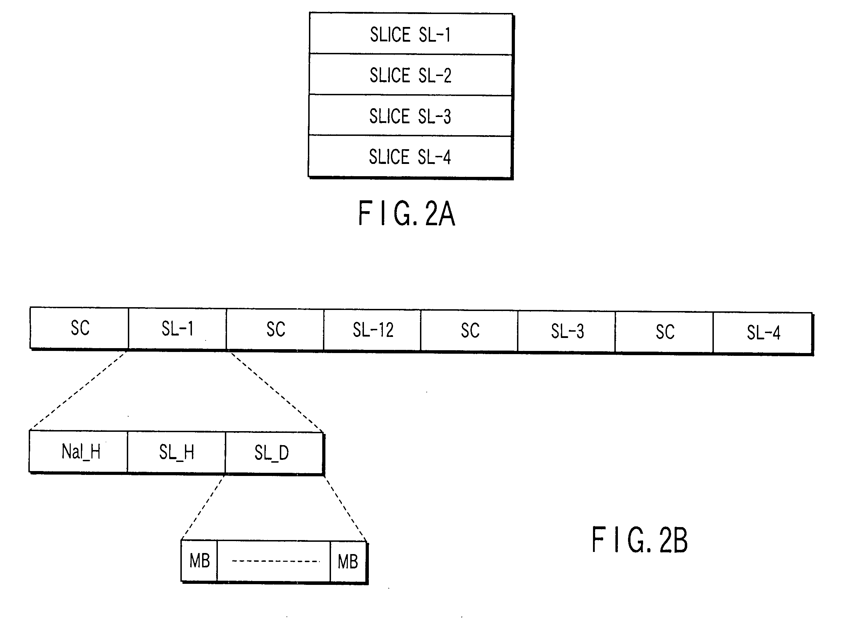

FIGS. 2A and 2B show a slice configuration of a frame of general moving picture data. As shown in FIG. 2A, a frame is constituted as an aggregation of one or a plurality of slices SL1 to SL4 corresponding to moving picture data. Here, one frame may comprise one slice, or one frame may comprise the plurality of slices SL-1 to SL-4 as shown in FIG. 2A. In the frame structure shown in FIG. 2A, one frame comprises four slices. A frame has a data structure comprising start codes SC (0x000001) written at the top of respective slices Sl-1 to SL4 and the slices SL-1 to SL-4 following the start codes SC as shown in FIG. 2B. In H.264 Baseline Profile, as types of the respective slices SL-1 to SL-4, there are three types, i.e., P-SLICE indicative of inter-frame coding, I-SLICE IDR (Instantaneous Decoding Refresh) indicative of refresh intra-frame coding, and I-SLICE Non-IDR indicative of scene change intra-frame coding.

In MPEG-4 Simple Profile, a VOP (Video Object Plane) is constituted as shown in FIG. 2A. There are two types, i.e., P-VOP indicative of inter-frame coding and I-VOP indicative of intra-frame coding. In the following description, a point to notice is that VOP is described as a frame since VOP is substantially equivalent to a frame.

It is to be noted that, in MPEG-4, a transmitter side utilizes this I-VOP indicative of intra-frame coding in both scene change and refresh. Additionally, as a timing for displaying a frame, PTS (Presentation Time Stamp) written in a header is utilized.

In H.264 Baseline Profile, each of the slices SL-1 to SL-4 comprises an NAL (Network Abstraction Layer) header Nal_H of one byte which follows the start code SC and slice data SL_D having picture coded data of a corresponding slice as shown in FIG. 2B. The slice data SL_D comprises an aggregation of macro blocks MB in which picture data in a slice is stored. In MPEG-4, likewise, VOP (Video Object Plane) comprises an aggregation of macro blocks MB.

In the NAL header Nal_H, nal_unit_type concerning a type of the NAL header Nal_H is written as syntax. Here, if nal_unit_type is 1 (nal_unit_type = 1), the slice SL corresponds to a non-IDR Slice (non-Instantaneous Decoding Refresh) which is either an I-slice or a P-slice whose decoding data is not immediately refreshed. Further, if nal_unit_type is 5 (nal_unit_type = 5), the slice SL corresponds to an IDR Slice (Instantaneous Decoding Refresh) whose decoding data is immediately refreshed.

If nal_unit_type is 1 or 5 (nal_unit_type = 1 or 5), each of the slices SL-1 to SL-4 includes between the NAL header Hal_H and the slice data SL_D a slice header SL_H in which a coding identifier indicative of a coding method of this slice is written. The slice header is indicative of a coding method of the slice. In slices of the same frame, picture data in the slices are coded by the same coding method. As types of this coding identifier, i.e., slice types, in H.264 Baseline Profile, there are P-SLICE indicative of the inter-frame coding, I-SLICE IDR indicative of the refresh intra-frame coding, and I-SLICE Non-IDR indicative of the scene change intra-frame coding.

A slice type (slice_type) is written in this slice header SL_H, and a type of the slice is determined based on this slice type (slice_type) and a type of the NAL header Nal_H (nal_unit_type). That is, if nal_unit_type is 1 (nal_unit_type = 1) and the slice type (slice_type) is 0 or 5 (slice_type = 0 or 5), the slice corresponds to P-slice. Furthermore, if nal_unit_type is 1 (nal_unit_type = 1) and the slice type (slice_type) is 2 or 7 (slice_type = 2 or 7), the slice corresponds to I-slice. It is to be noted that the slice SL corresponds to IDR Slice if nal_unit_type is 5 (nal_unit_type = 5), and hence the slice type (slice_type) is either 0 or 5 (slice_type = 0 or 5).

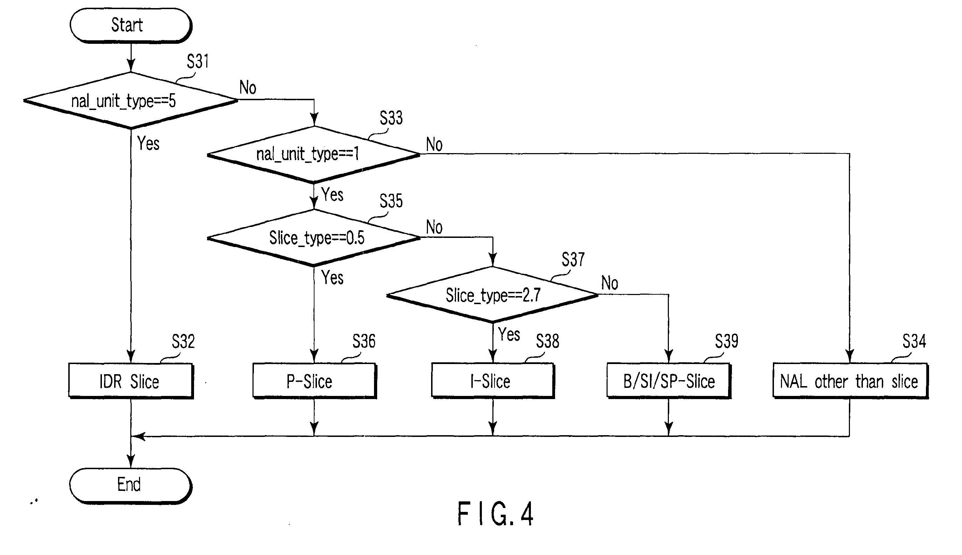

FIG. 3 is a block diagram schematically showing an apparatus which decodes a moving picture according to an embodiment of the present invention. As shown in FIG. 3, the decoding apparatus comprises a reception portion 10 which receives a coded stream, and an error detection portion 1 including a syntax analyzer (grammar analyzer) which analyzes a syntax included in frames in the coded stream and detects an error in the coded stream 10. Furthermore, the decoding apparatus also includes a coding identifier detection portion 2 which identifies a type of coding by detecting a coding identifier included in frames in the coded stream 10 and a decoding portion 3 which decodes encoded picture data into picture data in accordance with each frame. Moreover, the decoding apparatus comprises a concealment portion 4 which executes concealment processing with respect to picture data in a frame in accordance with each frame when picture data in each frame includes errors as will be described later in detail, a decoded picture buffer 5 which temporarily stores decoded frame picture data in each of a plurality of buffer areas BUF1 to BUF5, a frame memory 6 which stores picture data in accordance with each frame, and a picture output portion 7 which outputs a frame picture.

When the coded stream 10 having the data structure shown in FIG. 2B is input to the reception portion 10 depicted in FIG. 3, the error detection portion 1 first detects whether an error is included in the coded stream 10. That is, when a start SC is detected in the coded stream 10, a NAL header Nal_H and a slice header SL_H following this start code SC are analyzed. In this analysis, a bit string in the coded stream 10 is taken out, and this bit string is compared with codes in a code table (not shown). If there is no description of the bit string in any code in the code table, it is determined that an error is included in the coded stream 10, and processing of this coded stream 10 is suspended until a next start code is received. When the bit string matches with codes in the code table (not shown), a confirmation is made upon whether a value obtained by decoding this bit string is semantically and grammatically correct. For example, if a value obtained by decoding is 10 even though slice_type can take any of 0 to 9, it is determined that an error is generated, and processing of the coded stream 10 is suspended. As another example, if a slice type (slice_type) is a value other than 2 or 7 (slice_type = 2 or 7), e.g., slice_type = 0 even though nal_unit_type is 1 (nal_unit_type = 1), it is determined that a grammatical error is generated, and processing of the coded stream 10 is likewise suspended until a next start code is received.

The coding type detection portion 2 checks the NAL header Nal_H and the slice header SL_H, and a coding type of the slice is recognized in the decoding apparatus. That is, as shown in FIG. 4, the detection portion 2 checks whether the NAL header Nal_H has a description that nal_unit_type is 5 (nal_unit_type = 5) (step S31). If nal_unit_type is 5, it is determined that the slice SL is an IDR Slice (step S32). If nal_unit_type is not 5 at the step S31, whether the NAL header Nal_H has a description that nal_unit_type is 1 (nal_unit_type = 1) is checked (step S33). If nal_unit_type is not 1, it is determined that the slice SL is NAL other than Slice (step S34). If nal_unit_type is 1, whether the slice header SL_H has a description that the slice type (slice_type) is 0 or 5 (slice_type = 0 or 5) is checked at a step S35. If the slice type (slice_type) is 0 or 5 (slice_type = 0 or 5) at the step S35, it is determined that the slice is a P-slice (step S36). If the slice type (slice_type) is not 0 or 5 (slice_type = 0 or 5) at the step S35, a judgment is made upon whether the slice type (slice_type) is 2 or 7 (slice_type = 2 or 7) at a step S37. If the slice type (slice_type) is 2 or 7 (slice_type = 2 or 7), it is determined that the slice is an I-slice (step S38). If the slice type (slice_type) is not 2 or 7 (slice_type = 2 or 7) at the step S37, it is determined that the slice is one of B/SI/SP-slices (step S39).

As described above, the detection portion 2 shown in FIG. 3 judges a coding type of each slice.

The decoding portion 3 depicted in FIG. 3 decodes slice data included in frames in the coded stream 10 into picture data, and stores it in buffer areas BUF1 to BUF4 of a decoded picture buffer 5 (DPB). Here, the decoding portion 3 decodes slice data in accordance with coding types of slices by making reference to picture data stored in the decoded picture buffer 5 (DPB) and stores it in the decoded image buffer 5 as will be described later.

Moreover, if an error is included in frames, the concealment portion 4 reads picture data in which this error is generated, performs concealment processing depending on a coding type of this data by making reference to other picture data stored in the decoded picture buffer 5, restores the error in the picture data, and again stores the data in the buffer areas BUF1 to BUF4 of the decoded image buffer 5 as will be described later.

The frame memory 6 reads picture data from the decoded picture buffer 5, develops it into frames, and stores the picture data in accordance with each frame. Additionally, the frame memory 6 outputs a frame picture to the picture output portion 7 with a predetermined timing, the picture output portion 7 continuously displays the input frame picture data by using a single frame step function, and a user recognizes it as a moving picture. Each portion in the block depicted in FIG. 1 is controlled by a non-illustrated control CPU, and executes processing.

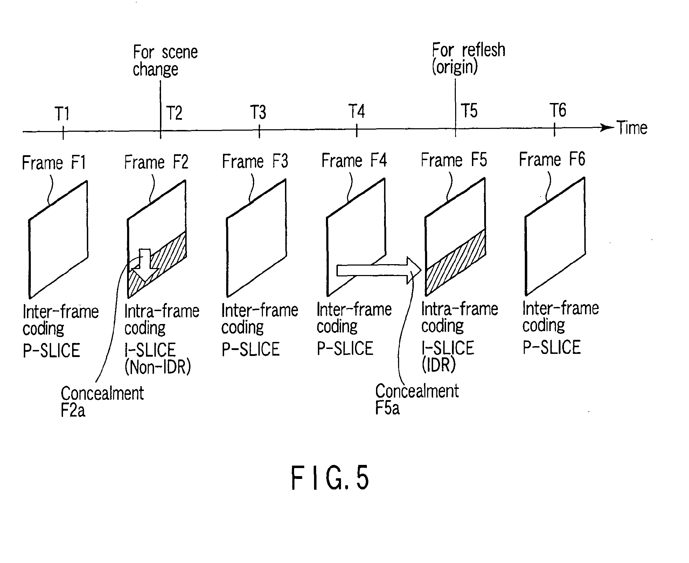

Concealment processing in the concealment portion 4 depicted in FIG. 3 will now be described with reference to FIG. 5.

FIG. 5 shows how frames F1 to F6 included in the coded stream 10 are sequentially input to the decoding apparatus illustrated in FIG. 3 with an elapse of time in a relationship between the time and frames. In FIG. 5, the frames F1, F3, F4 and F6 correspond to frames subjected to inter-frame coding (which will be referred to as P-frames hereinafter) and each of the P-frames F1, F3, F4 and F6 comprises a P-SLICE. Further, the frames F2 and F5 correspond to frames subjected to intra-frame coding (which will be referred to as I-frames hereinafter), and each of the I-frames F2 and F5 comprises an I-SLICE.

In an example shown in FIG. 5, the concealment processing will be described on the assumption that an error is generated in the I-SLICE of each of the frames F2 and F5 comprising the I-SLICE as indicted by hatching. Here, the frame F2 corresponds to a scene change I-frame (Non-IDR) and the frame F5 corresponds to an I-frame (IDR) which is periodically inserted for refresh.

Since the frame F2 is the scene change I-frame (Non-IDR), a picture content is greatly switched between the frame F1 and the frame F2, and a correlation between these frames is small. Therefore, a macro block in the vicinity of a macro block in which the error in the frame F2 is generated is utilized, and the macro block in which the error is generated is subjected to the concealment processing as indicated by reference character F2a.

In general, since the frame F5 is the I-frame, a neighboring macro block is utilized in the frame 5 having an error generated therein, and the macro block in which the error is generated is subjected to the concealment processing. In this embodiment according to the present invention, however, since the frame F5 is the I-frame (IDR) which is periodically inserted for refreshment, this frame is subjected to the concealment processing by utilizing a fact that the preceding frame F4 and the frame F5 have the strong correlation in case of the refresh frame even if this frame is the I-frame. That is, the macro block in which the error is generated in the frame F5 is subjected to the concealment processing by making reference to the frame F4 and utilizing a macro block in this frame F4 placed at the same position as the macro block in the frame F5 as indicated by reference character 5a.

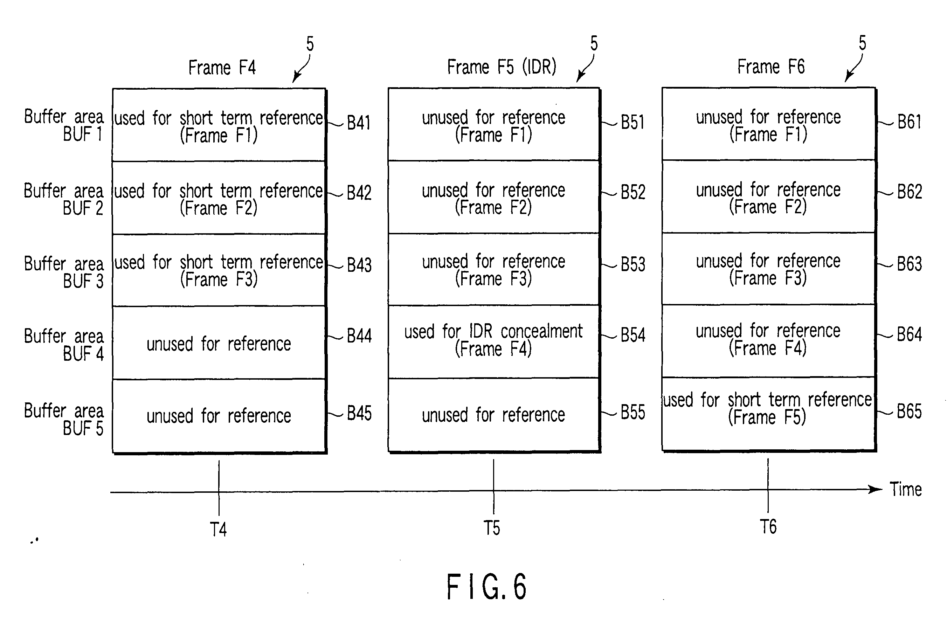

FIG. 6 is a schematic view showing a transition of reference frames stored in the decoded picture buffer 5 with an elapse of time. As shown in FIG. 6, the decoded picture buffer 5 includes buffer areas BUF1 to BUF5 for a plurality of frames, e.g., five frames. Decoded picture data of frames subjected to decoding and display is stored in the buffer areas BUF1 to BUF5 of the decoded picture buffer 5. Further, picture information indicative of whether reference is made to this decoded picture data in order to decode a next frame is also stored in the buffer areas BUF1 to BUF5. Here, as the picture information, there are "used for short term", "unused for reference" and "used for IDR concealment".

With a given a timing T4 before starting decoding of the frame F4, the frames F1 to F3 which are frames preceding the frame F4 in terms of time are combined into decoded picture data, and the obtained decoded picture data is stored in each of the buffer areas BUF1 to BUF3 as indicated by reference characters B41 to B43. Then, when the frame F4 is recognized as a P-frame which is subjected to inter-frame decoding, since reference should be made to the decoded picture data of the frames F1 to F3 in decoding of the frame F4, "used for short term reference" or "used for long term reference" is stored together with the decoded picture data of the frames F1 to F3 in the buffer areas BUF1 to BUF3 as indicated by reference characters B41 to B43.

With a timing T5 before starting decoding of the frame F5, the decoded picture data obtained by decoding the frame F4 is newly stored in the buffer area BUF4 as indicated by reference character B54.

In general, when the frame F5 is recognized as a refresh I-frame (IDR in H.264), since the frame F5 is subjected to intra-frame coding, all the decoded picture data of the frames F1 to F4 are information which is not referred in decoding of the frame F4, and hence "unused for reference" is set. In this embodiment, however, as indicated by reference character B54, in regard to the decoded picture data of the frame F4 immediately before the frame F5 as the refresh I-frame, "used for IDR concealment" indicative of restoration information is set to the frame F4 for the concealment processing in preparation for occurrence of an error in the frame F5. This restoration information "used for IDR concealment" is stored and kept together with the decoded picture data of the frame F4. This setting of "used for IDR concealment" is not restricted to the setting of the decoded picture data of the immediately preceding frame F4, and it may be set to the decoded picture data of any of the frames F1, F2, F3 and F4 as long as the setting target is any of other frames F1, F2 and F3 stored in the decoded picture buffer 5.

With a timing T6 before starting decoding of the frame F6, the frame F5 is decoded and the decoded picture data is newly stored in the buffer area BUF5 as indicated by reference character B65. Moreover, when the frame F6 is recognized as a P-frame, since this frame F6 is a P-frame immediately after the refresh frame F5, reference is made to the refresh frame F5 as an origin with a next timing T7 (not shown) following the timing T6, and the frame F6 is decoded. In preparation for decoding of the frame F6, "unused for reference" is set with respect to the decoded picture data of the frames F1 to F4 with the timing T6 as indicated by reference characters B61 to B64. Additionally, with the timing T6, the decoded picture data of the frame F5 is set as "used for short term reference" or "used for long term reference" as indicated by reference character B65. That is, in the processing according to this embodiment, at a point in time of the timing T5, information of "used for IDR concealment" is temporarily given in place of information of "unused for reference" as indicated by reference character B54. Further, at a point in time of the timing T6, information of "unused for reference" is rewritten from information of "used for IDR concealment" as indicated by reference character B64.

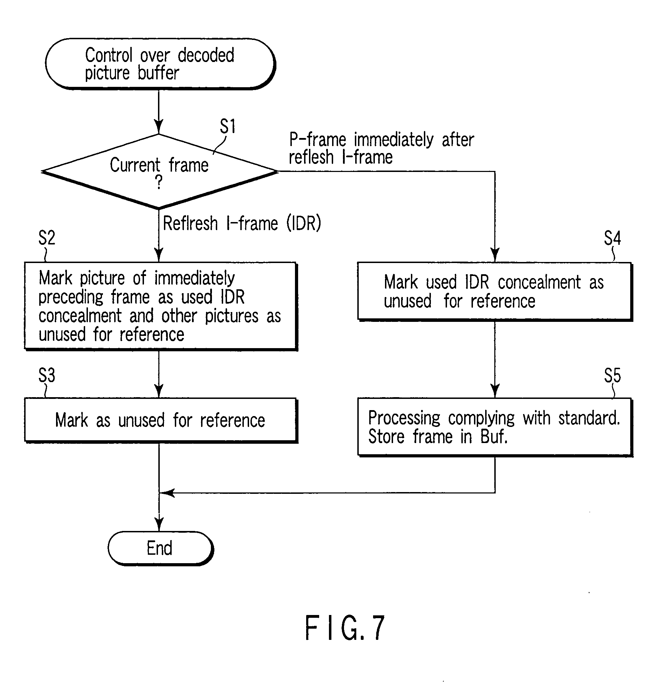

A description will now be given as to an operation of the decoded picture buffer with respect to the refresh I-frame F5 and the P-frame F6 following this I-frame F5 with reference to FIG. 7.

Before starting decoding of a frame, a coding type of this frame is first checked (step S1). If the coding type of the frame is the refresh I-frame (IDR in H.264) at the step S1, the decoded picture data of the immediately preceding frame F4 is set as "used for IDR concealment" (step S2). That is, at the step S2, the frame F5 at the timing T5 shown in FIG. 6 corresponds to the refresh I-frame, and the decoded picture data of the frame F4 is set as "used for IDR concealment" as indicated by reference character B54 in FIG. 6.

Furthermore, as indicated by reference characters B51, B52 and B53 in FIG. 6, "unused for reference" is set with respect to the decoded picture data of such frames having "used for short term reference" or "used for long term reference" as indicated by reference characters B41, B42 and B43 in FIG. 6, and reference is not made to these frames, namely, these frames are cleared (step S3).

Then, in decoding processing when the refresh I-frame F5 includes an error, if there is a reference frame F4 having information of "used for IDR concealment" in the decoded picture buffer, reference is made to this reference frame F4, and the refresh I-frame F5 is subjected to the restoration processing. In decoding processing when no error is included, since this processing is decoding of the I-frame F5, the reference frame F5 having information of "used for IDR concealment" is kept in the decoded picture buffer as it is without making reference to the preceding frame F4.

If the frame F6 which is started to be decoded at the step S1 is a P-frame immediately after the refresh I-frame F5, picture information having "used for IDR concealment" information in the decoded picture buffer is set as "unused for reference" (step S4). That is, at the step S4, the frame F6 at the timing T6 shown in FIG. 6 corresponds to the P-frame, and "used for IDR concealment" information indicated by reference character B54 in FIG. 6 is rewritten into "unused for reference" as indicated by reference character B64 in FIG. 6. Based on this processing, the decoded picture data of the frame F4 preceding the refresh I-frame F5 can be cleared. Thereafter, processing complying with the standard is executed (step S5).

A moving picture generally has characteristics that a correlation on the time base, i.e., a correlation between frames is stronger than a correlation in a frame. Based on such characteristics, concealment processing when an error is generated in a refresh I-frame can be further accurately executed, and a reproduced picture quality can be further improved.

In particular, in mobile-oriented broadcasting, an error mixing frequency is high, and approximately one refresh I-frame only is inserted every two seconds. Therefore, if the accuracy of the concealment processing for the refresh I-frame is low, all P-frames of the inter-frame coding following the refresh I-frame as an origin are adversely affected. The concealment processing of the refresh I-frame can be carried out with the high accuracy, and a reproduced picture quality can be improved.

Further, this processing can be effected by the simple processing shown in FIG. 7, and it can be readily carried out in a small device such as a mobile phone.

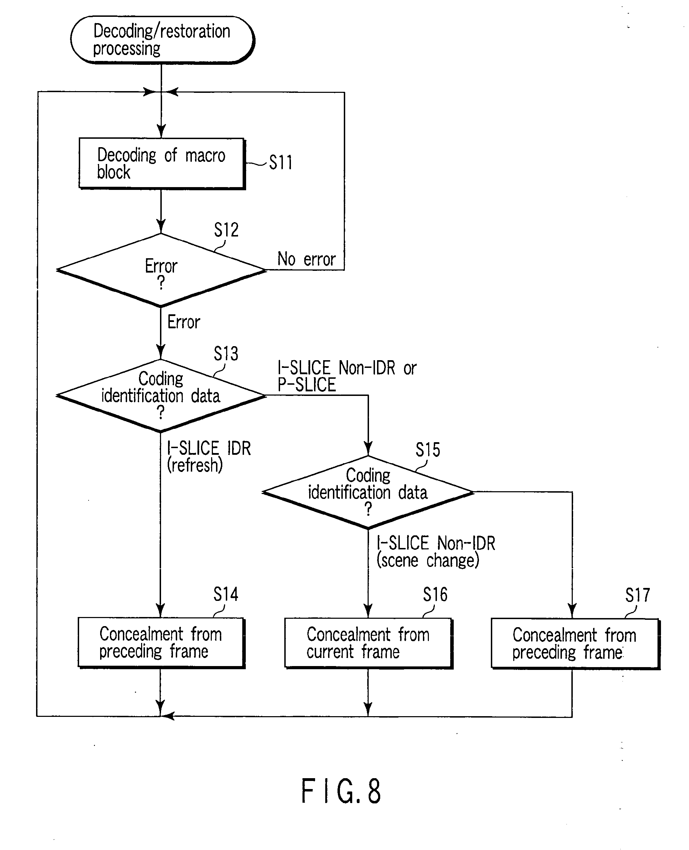

FIG. 8 is a flowchart illustrating an operation in the moving picture decoding apparatus depicted in FIG. 2, and shows a processing operation with respect to the data structure of H.264 depicted in FIGS. 2A and 2B.

In the decoding restoration processing after the operation which controls the decoded picture buffer 5 shown in FIG. 7, the decoding processing of picture data is executed in a macro block unit, and an error is detected (steps S11 and S12). If there is no error, a macro block included in a slice of a frame is subjected to decoding processing (step S11), the decoding processing of the macro block is continued, and picture data decoded in accordance with each macro block is sequentially stored in the decoded picture buffer 5.

If there is an error, a coding identifier of a slice including the macro block is checked as shown in FIG. 4 (step S13). Furthermore, if the coding identifier is I-SLICE IDR at the timing T5 shown in FIG. 6, the frame is a refreshment frame although it is a frame as a target of intra-frame coding. Therefore, it is determined that a correlation with a preceding frame is strong, and the concealment processing is executed from the preceding frame (step S14). As to this processing, if a reference frame which has information of "used for IDR concealment" (for IDR reference) and is denoted by reference character B54 in FIG. 6 exists in the decoded picture buffer described in conjunction with FIG. 6, the restoration processing is executed by using this reference frame.

If the coding identifier is I-SLICE Non-IDR (step S15), since this frame is a scene change frame, a correlation with a preceding frame is weak, and hence the concealment processing is executed from the current frame (step S16) .

If the coding identifier is P-SLICE (step S15), since this frame is an inter-frame coding frame, a correlation with a preceding frame is strong, and hence the concealment processing is executed by using the preceding frame (step S17).

With the above-described processing, it is possible to further accurately perform the concealment processing when an error is generated in I-SLICE IDR for refresh, thereby improving a reproduced picture quality.

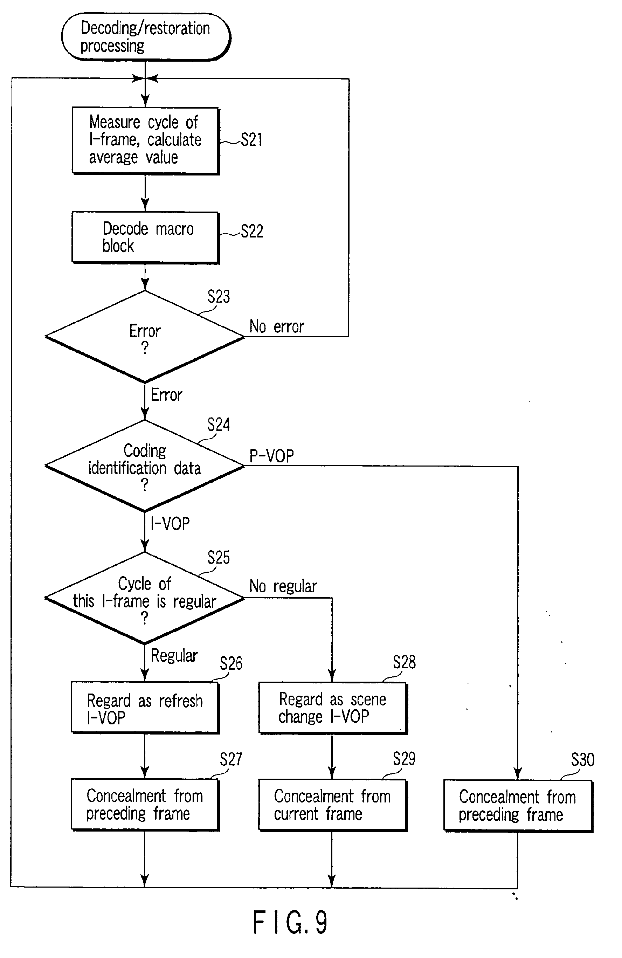

FIG. 9 is a flowchart illustrating an operation concerning another embodiment of the moving picture decoding apparatus shown in FIGS. 2. A description will be given as to an operation which processes data of MPEG-4 having such a structure as shown in FIGS. 2A and 2B after a control operation of the decoded picture buffer shown in FIG. 7 with reference to FIG. 9.

In MPEG-4, in regard to I-VOP, a transmission side including the coding apparatus recognizes and transmits one of I-VOP for refresh and I-VOP for scene change, but there is no difference between them in the coding identifier, and hence the coding apparatus side cannot directly discriminate. A reception side determines whether I-VOP is I-VOP for refresh or I-VOP for scene change in accordance with the following discrimination method.

First, the coding identifier is checked, and I-VOP or P-VOP is determined. In case of I-VOP, an interval of PTS (Presentation Time Stamp) attended to an I-frame to which this I-VOP belongs is measured for a plurality of number of times. For example, an average value of PTS for 10 times is calculated, and this average value is determined as an average cycle of the I-frame and stored (step S21). Although both I-VOP for refresh and I-VOP for scene change are mixed in the I-frame, the I-VOP for refresh has a higher frequency than that of the I-VOP for scene change because of characteristics of a moving picture. Therefore, if the average value of PTS for 10 times corresponding to an average value of 10 cycles can be obtained, a cycle of the refresh I-frame which is cyclically transmitted is substantially determined.

Simultaneously with this processing, the regular decoding processing (step S22) and the error detection (step S23) are executed.

If existence of an error is detected in the error detection (step S23), whether VOP is I-VOP or P-VOP is checked (step S24). If it is I-VOP, a difference in time between this I-frame to which this I-VOP belongs to and a preceding I-frame is measured. A judgment is made upon whether this time difference is substantially the same as the average value calculated at the step S21 (step S25). If they are substantially the same, the I-VOP is regular, namely, the I-VOP is regarded as I-VOP for refresh (step S26). The processing by which the I-VOP is regarded as the I-VOP for refresh corresponds to processing at the timing T5 shown in FIG. 6. Further, it is determined that the frame of this I-VOP has a strong correlation with a preceding frame, and this frame is subjected to the concealment processing from the preceding frame (step S27). As described in conjunction with reference character B54 in FIG. 6, if a reference frame having information of "used for IDR concealment" exists in the decoded picture buffer, the restoration processing is executed by using this reference frame.

At the step S25, if a difference in time between this I-frame and the preceding I-frame is largely different from the average value calculated at the step S21, the I-VOP is regarded as I-VOP for scene change (step S28). In case of the I-VOP for scene change, the frame of this I-VOP has a weak correlation with a preceding frame, the concealment processing is executed from the current frame (step S29).

If VOP is P-VOP at the step S24, since this P-VOP is VOP for inter-frame coding, its frame has a strong correlation with a preceding frame. Therefore, the frame of this VOP is subjected to the concealment processing by making reference to the preceding frame (step S30).

With the above-described processing, it is possible to further accurately perform the concealment processing when an error is generated in the I-VOP for refreshment, thereby greatly improving a reproduced picture quality.

It is to be noted that, at the step S21, as another method, a cycle of the I-frame may be calculated for a plurality of number of times, the cycles may be subjected to statistical processing, and a most frequently appeared cycle may be recognized as a cycle of the refresh I-frame. Moreover, if a cycle of the refresh I-frame is previously defined and identified, it may be stored as a defined value.

As described above, according to the moving picture decoding apparatus and the moving picture decoding method of the present invention, the simple concealment processing which can be used in a small device such as a mobile phone is executed in addition to the concealment processing at the time of occurrence of an error which is defined in H.264 or the like, and a reproduced picture quality can be further improved.

## Claims

1. An apparatus which decodes a moving picture **characterized by** comprising:
a reception portion (10) configured to receive a coded stream including a scene change I-frame coded in a frame, a first identifier for the scene change I-frame, a first P-frame coded between frames, a second identifier for the first P frame, a refresh I-frame coded in a frame following the first P-frame and a third identifier for the refresh I-frame;
a coding identifier detection portion (2) configure to detect the first, second and third identifiers, and discriminate the scene change I-frame, the P-frame and the refresh I-frame;
a decoding portion (3) configured to decode the scene change I-frame into first frame picture data in response to detection of the first identifier, decode the first P-frame into second frame picture data based on the I-frame picture data in response to detection of the second identifier, and decode the refresh I-frame into third frame picture data in response to the third identifier;
a buffer portion (5) having first, second and third buffer areas in which the first, second and third frame picture data and first, second and third reference information are respectively stored; and
a restoration processing portion (1, 3, 4, 5) configured to set the second frame picture data for restoration if there is an error in the refresh I-frame when decoding the refresh I-frame, restores the refresh I-frame by making reference to the second frame picture data, and store it as the third frame picture data in the third buffer area.

2. The apparatus according to claim 1, **characterized in that** information that the first frame picture data is non-reference data is written in the first reference information and information that the second frame picture data is restoration data is written in the second reference information in response to detection of the third identifier.

3. The apparatus according to claim 2, **characterized in that** information in the second reference information is changed to information that the second frame picture data is non-reference data and information that the third frame picture data is reference data is written in the third reference information after decoding into the third frame picture data.

4. The apparatus according to claim 1, **characterized in that** the detection portion (2) measures a cycle of a frame subjected to the intra-frame coding, and determines that the frame is a refresh frame if the cycle is substantially the same as a predefined cycle of the refresh frame.

5. An apparatus which decodes a moving picture **characterized by** comprising:
a reception portion (10) configured to receive a coded stream including a scene change I-frame coded in a frame, a first identifier for the I-frame, a first P-frame coded between frames, a second identifier for the first P frame, a refresh I-frame coded in a frame following the first P-frame and the first identifier for the I-frame;
a detection portion (2) configured to detect the first and second identifiers and discriminate the I-frame and the P-frame, the detection portion (2) measuring a cycle of the I-frame for an arbitrary number of times and judging the refresh I-frame based on the measured cycles;
a decoding portion (3) configured to decode the scene change I-frame into first frame picture data in response to the first identifier, decodes the first P-frame into second frame picture data based on the I-frame picture data in response to detection of the second identifier, and decode the refresh I-frame into third frame picture data in response to the first identifier;
a buffer portion (5) having first, second and third buffer areas in which the first, second and third frame picture data and first, second and third reference information are respectively stored; and
a restoration processing portion (1, 3, 4, 5) configured to set the second frame picture data for restoration if there is an error in the refresh I-frame when decoding the I-frame which is determined as the refresh I-frame in the detection portion (2), restores the refresh I-frame by making reference to the second frame picture data, and stores it as the third frame picture data in the third buffer area.

6. The apparatus according to claim 5, **characterized in that** the detection portion (2) calculates an average value of the cycle from the measured cycles, and determines as the refresh I-frame an I-frame whose cycle is substantially the same as the average value.

7. The apparatus according to claim 1, **characterized in that** the detection portion (2) calculates a most frequently generated cycle from the measured cycles, and determines as the refresh I-frame an I-frame whose cycle is substantially the same as the most frequently generated cycle.

8. A method for decoding a moving picture **characterized by** comprising:
receiving a coded stream including a scene change I-frame coded in a frame, a first identifier for the scene change I-frame, a first P-frame coded between frames, a second identifier for the first P-frame, a refresh I-frame coded in a frame following the first P-frame, and a third identifier for the refresh I-frame;
detecting the first, second and third identifiers, and discriminating the scene change I-frame, the P-frame and the refresh I-frame;
decoding the scene change I-frame into first frame picture data in response to detection of the first identifier, decoding the first P-frame into second frame picture data based on the I-frame picture data in response to detection of the second identifier, and decoding the refresh I-frame into third frame picture data in response to the third identifier;
storing the first, second and third frame picture data and first, second and third reference information in first, second and third buffer areas, respectively; and
setting the second frame picture data for restoration if there is an error in the refresh I-frame when decoding the refresh I-frame, restoring the refresh I-frame by making reference to the second frame picture data, and storing it as the first frame picture data in the third buffer area.

9. The method according to claim 8, **characterized in that** information that the first frame picture data is non-reference data is written in the first reference information and information that the second frame picture data is restoration data is written in the second reference information in response to detection of the third identifier.

10. The method according to claim 9, **characterized in that** information in the second reference information is changed to information that the second frame picture data is non-reference data and information that the third frame picture data is reference data is written in the third reference information after decoding into the third frame picture data.

11. The method according to claim 9, **characterized in that** the detection measures a cycle of a frame subjected to the intra-frame coding, and determines the frame as a refresh frame if the cycle is substantially the same as a predefined cycle of the refresh frame.

12. A method for decoding a moving picture **characterized by** comprising:
receiving a coded stream including a scene change I-frame coded in a frame, a first identifier for the I-frame, a first P-frame coded between frames, a second identifier for the first P-frame, a refresh I-frame coded in a frame following the first P-frame, and the first identifier for the I-frame;
detecting the first and second identifiers and discriminates the I-frame and the P-frame, measuring a cycle of the I-frame for an arbitrary number of times, and determining the refresh I-frame based on the measured cycles;
decoding the scene change I-frame into first frame picture data in response to detection of the first identifier, decoding the first P-frame into second frame picture data based on the I-frame picture data in response to detection of the second identifier, and decoding the refresh I-frame into third frame picture data in response to the first identifier;
storing the first, second and third frame picture data and first, second and third reference information in first, second and third buffer areas, respectively; and
setting the second frame picture data for restoration if there is an error in the refresh I-frame when decoding the I-frame determined as the refresh I-frame in the detection portion (2), restoring the refresh I-frame by making reference to the second frame picture data, and storing it as the third frame picture data in the third buffer area.

13. The method according to claim 12, **characterized in that** the detection calculates an average value of the cycle from the measured cycles, and determines as the refresh I-frame an I-frame whose cycle is substantially the same as the average value.

14. The method according to claim 12,
**characterized in that** the detection calculates a most frequently generated cycle from the measured cycles, and determines as the refresh I-frame an I-frame whose cycle is substantially the same as the most frequently generated cycle.
